# EUROPEAN PATENT SPECIFICATION

(11) **EP 1 682 154 B1**
(45) Date of publication and mention of the grant of the patent: **04.08.2010**
(21) Application number: 03819046.8
(22) Date of filing: 24.10.2003
(51) Int. Cl.: A61K 31/56, C07J 9/00, A23L 1/30

(54) **PROCESS FOR PREPARING PHYTOSTEROL DISPERSIONS FOR APPLICATION IN BEVERAGES**
VERFAHREN ZUR HERSTELLUNG VON PHYTOSTEROL-DISPERSIONEN ZUR VERWENDUNG IN GETRÄNKEN
PROCEDE DE PREPARATION DE DISPERSIONS DE PHYTOROSTEROL POUR UTILISATION DANS DES BOISSONS

(43) Date of publication of application: 26.07.2006
(73) Proprietor: The Coca-Cola Company, Atlanta, GA 30313 (US)
(72) Inventor: LERCHENFELD, Erich, P., Altamonte Springs, FL 32714 (US); STRIEGEL, Donald, E., Clermont, FL 34711 (US)
(74) Representative: Gordon, Kirsteen Helen
(86) International application number: PCT/US2003/033950
(87) International publication number: WO 2005/049037

(56) References cited:
- WO-A-00/41491
- WO-A-00/45684
- WO-A-01/37681
- WO-A-03/086108
- US-A- 5 445 811

## Description

### BACKGROUND OF THE INVENTION

### Field of the Invention

The invention relates to aqueous compositions, such as beverages, containing plant sterols for human and veterinary use and processes for their manufacture. Typical beverages include fruit and vegetable juices. Other typical beverages include sports beverages, drinks, or beverages employed to restore electrolytes lost due to illness. Further typical beverages include carbonated beverages including soft drinks and so-called "botanical flavor" drinks such as cola and other natural and artificial flavor drinks.

### RELATED ART

Researchers have investigated methods of preventing atherosclerosis, one of the underlying causes of cardiovascular disease, and have evidence that cholesterol plays a role in this disease by contributing to the formation of atherosclerotic plaques in blood vessels, causing interference with blood circulation to the heart muscles, kidneys, brain, and limbs. Some data show that a 1% reduction in a person's total serum cholesterol yields a 2% reduction in risk of coronary artery disease and a 10% decrease could prevent about 100,000 deaths in the United States annually. As early as 1953, the scientific literature reported that plant sterols have some effect in reducing atherosclerotic events in mammals, reduction in blood serum cholesterol in man, and the reduction of serum cholesterol in young men with atherosclerotic heart disease. (Pollak, Circulation 7, 696-701; 702-706 (1953); Farquhar et al., Circulation, 14, 77-82 (1956)). Other scientific literature establishes that plant sterols and stanols do, in fact, lower the level of serum cholesterol in humans; however, because of poor solubility in water, it was difficult to prepare products suitable for human and veterinary consumption that contained these plant sterols or stanols.

For the most part, plant sterols or stanols were employed in margarines and other so-called spreads or similar food products because of their hydrophobic properties. United States Patents Nos. 3,881,005 and 4,195,084, both assigned to Eli Lilly, describe grinding or milling plant sterols in order to enhance their solubility. Eli Lilly at one time marketed a sterol preparation from tall oil and later from soybean oil under the trademark Cytellin that lowered serum cholesterol by about 9%. Kuccodkar et al., Atherosclerosis, 23: 239-248 (1976). The product, however, never received widespread consumer acceptance.

Fruit juice-containing products, i.e., aqueous-based beverages and preparations containing fruit juice (as well as concentrates for preparing such beverages and products), are used in the art, and have achieved a relatively high degree of commercial acceptance. The incorporation of hydrophobic ingredients into these products presents a difficulty well known in the art since hydrophobic ingredients have a different density than water and as a result, at the time of purchase and consumption of the product, a hydrophobic component may separate and float to the surface or sink to the bottom. For example, the hydrophobic component that floats to the surface produces undesirable "ringing," which is found in beverages, such as juices containing a hydrophobic ingredient with a density less than water, and results in a product that is non-uniform throughout the container.

Fruit juice-containing products packaged in transparent or translucent (e.g., glass or plastic) containers must avoid this separation since aesthetically undesirable visible separation of the product impacts on consumer acceptance. Agitation of the fruit juice-containing product in its container prior to use provides a temporary dispersion of the hydrophobic ingredient, however, this only amounts to a short term solution, as the hydrophobic ingredient can separate again following agitation. Hydrophobic, fat-soluble or oleophilic ingredients, including vitamins, oils, extracts, flavors, and sterols, when added to fruit juice-containing products require special treatment to ensure incorporation either by suspension or dispersion into the fruit juice-containing product so that they will not separate.

Prior art attempts to overcome these difficulties typically make use of several methods including homogenization, encapsulation, and/or the addition of stabilizers, gums, emulsifiers, and the like; however, these methods increase the cost of the product, and in some instances are illegal in certain standardized products such as, a citrus juice, e.g., orange juice. The consumer may also find some of these products undesirable from a labeling, texture, and viscosity standpoint. Stabilizers and gums often add viscosity, i.e., thickness to a fruit juice-containing product, thereby detracting from its organoleptic impression. Additionally, dispersing plant sterols in juices or drinks causes the beverage to have a powdery texture, which also impacts negatively on consumer acceptance.

Because of consumer recognition and acceptance, some juice beverages should maintain a turbid appearance and should not produce a ring at the surface of the juice when in the container or a glass, making it necessary to provide a fruit juice and/or fruit juice concentrate containing hydrophobic materials in a stable dispersion. Consumer recognition and acceptance of turbidity in some fruit juice products such as citrus juices, e.g., orange juice and other beverage products, requires stability of the product for this reason, both during refrigeration or the shelf life of the product, as well as at the point of consumption.

Tiainen et al., U. S. Patent No. 6,129,944, describes a method for producing a product containing a plant sterol by forming a homogeneous suspension of a microcrystalline plant sterol and a sweetening agent in an aqueous solution.

Vulfson et al., WO 00/41491, discloses hydrophobic compounds such as plant sterols and lycopenes as supplements to food products and beverages such as oleomargarine products, drinks, soups, sauces, dips, salad dressings, mayonnaise, confectionery products, breads, cakes, biscuits, breakfast cereals, and yogurt type products. Vulson et al., in combining the plant sterol or lycopene with the food product, theorizes that the food product, which has both hydroxyl and carboxyl groups, interacts with the surface of the sterol or lycopene.

The reference goes on to describe producing a fine suspension of plant sterols in water in the absence of surfactants and without grinding the plant sterol with sugars, as disclosed United States Patents Nos. 3,085,939; 4,195,084; 3,881,005; and GB 934,686. Vulfson et al. by contrast, forms a suspension or slurry of plant sterols in water at from about 10% to about 30% (by weight) of sterol by extensive homogenization using conventional methods and a small volume of a concentrated aqueous solution of the food product, which the inventors describe as a "coating material."

Haarasilta et al., WO 98/58554, describes a premix used in the food industry containing a pulverized plant sterol and a conventional foodstuff ingredient such as fruit, vegetable, or berry type of material, particularly in a powder form and methods for preparing the premix. Grinding the plant sterol and the foodstuff such as berries, fruits, or vegetables according to methods and devices disclosed in Finnish patent applications FI 963 904 and FI 932 853 and with a grinder operating on the so-called impact milling principle, such as an Atrex mill manufactured by Megatrex Oy, produce this result. The inventors note that when applying the process of the invention to cereal in combination with a plant sterol, the temperature of the cereal grains rises due to the effect of mechanical energy on the grains, thereby providing heat treatment of the grains in conjunction with grinding.

Zawistowski, WO 00/45648, describes a method of preparing microparticles of plant sterols and plant stanols or mixtures of both by dispersing and suspending the plant sterols and plant stanols in a semi-fluid, fluid, or viscous vehicle and exposing the vehicle so formed to impact forces. The method involves dispersing or otherwise suspending the plant sterol and/or plant stanol in a suitable semi-fluid, fluid, or viscous vehicle followed by applying impact forces to the vehicle to produce microparticles. Zawistowski develops these impact forces by creating high-shear either with an air-atomization nozzle, a pneumatic nozzle, a high-shear mixer, or colloid mill, but preferably a microfluidizer commercially available from Microfluidics Incorporation, Newton, Massachusetts.

Zawistowski observed that the plant sterols and/or plant stanols prepared in this way have greater "solubility" not only in oil based delivery systems but also in other media and can be incorporated into beverages such as colas, juices or dietary supplement and/or milk replacement drinks.

Gottemoller, WO 01/37681 A1, also describes a process of combining a plant sterol and/or plant stanol with a water-soluble protein and optionally an emulsifier by grinding the plant sterols and plant stanols or prilling it to produce a powdered product before adding it to an aqueous material.

Tarr et al., WO 94/27451, describes a process for making a thickener from citrus fruit for beverages by preparing a slurry of water and citrus pulp having a solids content of from 0.15% to 10% by weight (anhydrous) followed by heating the slurry to a temperature from 70° C to 180° C (158° F to 356°F) for 2 to 240 minutes, and subjecting the slurry to high shear treatment at a shear rate of from 20,000 sec⁻¹ to 100,000,000 sec⁻¹ by homogenization at a pressure of from 1,000 psig to 15,000 psig (6.9 to 103.4 MPa above atmospheric) and colloidal milling.

It would be an advantage to overcome at least one of the difficulties in the related art. At least one of these other advantages are realized according to the present invention, which provides a process for producing a substantially stable dispersion consisting essentially of a hydrophobic plant sterol and an aqueous material such as an aqueous beverage concentrate, and products made by this process, all of which substantially obviate one or more of the limitations or disadvantages of the processes and compositions of the related art without, for example, increasing viscosity, imparting off-flavors, or a powder taste, introducing undesirable ingredients, or producing an undesirable visual appearance.

The specification sets out additional features and advantages which may be realized by the invention, which in part, a skilled artisan will fined apparent from the description and may learn by practice of the invention, and who will realize the objectives and other advantages of the invention obtained by the process and composition particularly pointed out in the written descriptions and claims hereof.

### SUMMARY OF THE INVENTION

To achieve at least one of these or other advantages and in accordance with the purpose of the invention, as embodied and broadly described, the inventors have found a process for producing a substantially stable dispersion comprising from 1 to 100 grams per liter of at least one hydrophobic plant sterol and an aqueous material comprising at least one fruit juice or fruit juice concentrate, wherein the solids content of said aqueous material is from 200 to 1000 grams per liter and wherein said plant sterol does not separate from said dispersion over a period of time from formation of said dispersion to 12 months after formation, after subjecting the dispersion to heating and cooling cycles and/or shelf storage during this time, comprising:
mixing the at least one hydrophobic plant sterol with said aqueous material to form a first dispersion of particles of the at least one hydrophobic plant sterol and said aqueous material; and
homogenizing the first dispersion to obtain a second dispersion of particles of the at least one hydrophobic plant sterol and said aqueous material, wherein the particle size of said hydrophobic plant sterol particles in said first dispersion is from 0.1 microns to 100 microns, or the particle size of said hydrophobic plant sterol particles in said second dispersion is from 0.1 microns to 100 microns, or wherein the particle size of said hydrophobic plant sterol particles in both said first dispersion and said second dispersion is from 0.1 microns to 100 microns,
wherein said first dispersion is not heated prior to homogenizing, and
with the proviso that said substantially stable dispersion does not contain any added emulsifiers or thickening agents.
Unless otherwise indicated, the term "plant sterol", as used in this specification and claims, is intended to include both a plant sterol and a plant stanol.

The process of the invention and resultant composition do not require the use of gums and/or emulsifiers in order to obtain a stable dispersion of the plant sterols in the aqueous material without separation, flavor impact and texture impact, especially in the manufacture of juice concentrates, such as citrus juice concentrates, e.g., orange juice concentrates.

A substantially stable dispersion according to the invention comprises a dispersion of a hydrophobic plant sterol in an aqueous material produced according to the process of the invention that results in a dispersion where the plant sterol does not separate from it over a period of time up to about 12 months, after subjecting the dispersion to several heating and cooling cycles and/or shelf storage during this time. When employed in citrus juice concentrates, if the plant sterol settles, it settles with the sinking pulp, which is a natural occurrence for citrus juices.

Citrus beverages such as orange juice have two types of pulp, one floats and the other sinks. Pulp that gives orange juice its cloud comprises a sinking pulp whereas floating pulp rises to the surface of the juice and the container. Plant sterols have a density lower than water and as a result will float to the top of an aqueous beverage such as a citrus beverage concentrate or juice. If not properly dispersed, the plant sterol will form a white ring at the top of the citrus beverage. One of the advantages of the present invention comprises providing a substantially stable dispersion of the plant sterol and aqueous material such as a citrus beverage concentrate or citrus beverage, with no separation of the plant sterol in a manner to form white rings at the top of the beverage. The inventors have found that the plant sterols of the present invention, which comprises the plant sterol in combination with a fruit juice concentrate, citrus juice concentrate, or beverage such as orange juice, may not rise to the surface of the beverage, but rather may remain dispersed in the beverage and causes an increase in the volume of sinking pulp at the bottom of the beverage. The increased volume of sinking pulp suggests the presence of the plant sterol in the sinking pulp. In any event, according to the invention, the fruit juice, citrus beverage concentrate, or citrus beverage formulated according to the present invention may be substantially free if not totally free of plant sterols that float to the surface.

A novel discovery made is that the process of the present invention does not require heating of the first dispersion before homogenizing or of the second dispersion after homogenizing. However, heating may be desirable for pasteurization and to prevent microbial spoilage of a product made by the process of the present invention. Thus, the process of the present invention may optinally further comprise one or more heating steps. In one optional embodiment, the process may further comprise a heating step wherein the first dispersion of particles of the hydrophobic plant sterol and the aqueous material are heated to a temperature of from about 43°C to about 100°C (about 110°F to about 212°F) for a period of time of from about 1 second to about 20 seconds to form a heated first dispersion. In another optional embodiment, the optional heating step may be performed after homogenizing.

In a further embodiment, forming the dispersion of the invention optionally includes cooling the optionally heated first dispersion to a temperature of from about 22°C (about 72°F) up to about 71°C (about 160°F) for a period of time of from about 1 second to about 12 seconds prior to homogenizing.

In yet another embodiment, the process may involve conducting the homogenizing at different pressures and multiple steps, and optionally at different temperatures of from about 22°C (about 72°F) up to about 71°C (about 160°F).

In a further embodiment of the invention, the aqueous material may comprise an aqueous beverage concentrate such as a fruit juice concentrate, e.g., a citrus juice concentrate such as an orange juice concentrate.

The invention also relates to the discovery that a dispersion of a hydrophobic plant sterol in an aqueous material may avoid the prior art difficulty of imparting a powdery taste to the dispersion, when the particle size of the hydrophobic plant sterol particles is from about 0.1 micron to about 50 microns, or the majority of hydrophobic plant sterol particles within this range will be from about 0.2 microns to about 10 microns, or in any event will substantially follow a bell curve distribution, for any of these particle size distributions. The term "majority" as used in this specification and claims means greater than 50%.

### BRIEF DESCRIPTION OF THE DRAWINGS

Fig. 1 is an example of the particle size distribution of a sample of micronized plant sterol having a total plant sterol content of greater than about 93%, and composed of β- sitosterol, β-sitostanol, campesterol, campestanol, stigmasterol, spinosterol, avenasterol, and brassicasterol, the mixture having a melting point of about 138°C. to about 141°C., derived from vegetable oils and tall oils, and supplied by MB Multi Bene Health Oy Ltd. of Finland.

Fig. 2 shows the volume distribution of the micronized plant sterol of Fig. 1.

### DETAILED DESCRIPTION

Accordingly, the invention relates to a process for producing a substantially stable dispersion comprising at least one hydrophobic plant sterol and an aqueous material such as an aqueous beverage concentrate wherein the at least one plant sterol is selected from plant sterols and plant stanols, and wherein the dispersion does not contain any added emulsifiers and thickening agents and other so-called "manufacturing aides, used in the food arts, e.g., encapsulation materials.

In mixing the hydrophobic plant sterol with the aqueous material such as an aqueous beverage concentrate to form a first dispersion and/or a second dispersion of particles of the at least one hydrophobic plant sterol and the aqueous material, the particle size of the at least one hydrophobic plant sterol particles in the first dispersion and/or the second dispersion is from about 0.1 microns to about 100 microns, or about 0.1 microns to about 50 microns, or from about 0.1 microns to about 30 microns, or from about 0.1 micron to about 10 microns, and in one embodiment will substantially follow a bell curve distribution. In another embodiment, in order to substantially avoid a powdery taste in the aqueous material, or concentrate, or beverage, the particle size of the at least one hydrophobic plant sterol particles in the first dispersion and/or the second dispersion is from about 0.1 micron to about 30 microns, or where the majority of particles will range in size in any one of the foregoing ranges of from about 0.2 microns to about 10 microns, or about 0.2 microns to about 2.5 microns, or about 0.4 microns to about 1.5 microns, or about 0.3 microns to about 0.4 microns, and in a further embodiment will substantially follow a bell curve distribution. All of the foregoing particle sizes and particle size ranges may also vary from plus or minus about 30%, or plus or minus about 20%, or plus or minus about 10%. Hydrophobic plant sterol particles of all of the above mentioned sizes and size ranges are well known in the art and may be obtained through commercial suppliers such as Cargill Co.

One aspect of the invention comprises a process for producing a substantially stable dispersion comprising at least one hydrophobic plant sterol and an aqueous material, wherein said plant sterol is selected from plant sterols and plant stanols comprising:
(a) mixing said hydrophobic plant sterol with said aqueous material to form a first dispersion of particles of said hydrophobic plant sterol and said aqueous material; and
(b) homogenizing said heated mixture to obtain a second dispersion of particles of said hydrophobic plant sterol and said aqueous material, wherein the particle size of said hydrophobic plant sterol particles in said first dispersion is from about 0.1 microns to about 100 microns, or the particle size of said hydrophobic plant sterol particles in said second dispersion is from about 0.1 microns to about 100 microns, or wherein the particle size of said hydrophobic plant sterol particles in both said first dispersion and said second dispersion is from about 0.1 microns to about 100 microns.

In one embodiment, the process may further comprise heating said first dispersion before the homogenizing step to form a heated first dispersion before homogenizing. In another embodiment, the process may also optionally comprise heating said second dispersion to form a heated second dispersion. In yet another embodiment, the process may optionally comprise heating both the first dispersion and second dispersion.

Another aspect of the process of the present invention is that it may produce a composition of matter which is a substantially stable dispersion of at least one hydrophobic plant sterol or plant stanol and an aqueous material which, in order to substantially avoid a powdery taste in the aqueous material, or concentrate, or beverage comprises hydrophobic plant sterol or plant stanol particles as described above. The composition of matter having this particle size, or particle size and particle size distribution to substantially avoid a powdery taste in the aqueous material, or concentrate, or beverage product, may be made according to the process of the invention, or by any other process used in the food arts, so long as it contains the plant sterol or plant stanol that has the particle size, or particle size and particle size distribution that avoids the powdery taste in the finished product, and, when not manufactured according to the process of the invention, may optionally contain manufacturing aids used in the food arts. These manufacturing aids may be used in amounts of from about 0.001 % by wt. to about 50% by weight of the plant sterol, or from about 0.01% by wt. to about 30% by weight of the plant sterol, or from about 0.01% by wt. to about 25 % by weight of the plant sterol or plant stanol, or from about 0.1% by wt. to about 20% by weight of the plant sterol or plant stanol, all based on the aqueous material, or concentrate, or beverage product.

The so-called "manufacturing aids" include encapsulation aids, starches, and gums used as thickening agents employed in the food arts, and pectin, de-methylated pectin and other pectin derivatives used in the food arts. The emulsifiers comprise modified food starches and other similar food-type emulsifiers whereas the gums include gum Arabic, seaweed extracts, alginates, plant or seed gums such as guar gum, or animal derived products such as gelatin as well as xanthan gum, locust gum, carrageenan, and the like. In addition to gum Arabic, other water soluble gums employed comprise angico gum, cebil gum, mesquite gum, cedar gum, and Indian gum, whereas the gums slightly soluble in water include tragacanth, sterculia, hog gum, amrad gum, and satinwood gum or gums that swell in water such as cherry gum, Sonora gum, or sassa gum. Other gums included in this aspect of the invention are defined in Hackh's Chemical Dictionary, 3d Ed., p. 392.

Scientific literature describes at least 44 plant sterols, and the skilled artisan can select any plant sterol from those that are available when practicing the present invention. The present invention, also involves using some of the plant sterols employed in the art. Some plant sterols in this regard include sitosterol, campesterol, stigmasterol, spinosterol, taraxasterol, brassicasterol, desmosterol, chalinosterol, poriferasterol, clionasterol, and ergosterol. The invention also employs mixtures of plant sterols, such as two component, three component, and four component mixtures.

The source of these and other plant sterols are rice bran, corn bran, corn germ, wheat germ oil, corn oil, safflower oil, oat oil, olive oil, cotton seed oil, soybean oil, peanut oil, black tea, green tea, colocsia, kale, broccoli, sesame seeds, shea oils, grapeseed oil, rapeseed oil, linseed oil, canola oil, tall oil and other oils obtained from wood pulp.

Plant sterols may also be hydrogenated to produce plant stanols. Accordingly, the plant stanols of the present invention are described as the hydrogenation products of the various plant sterols such as sitosterol but may also be obtained naturally from various plants used in the art, without hydrogenating the plant sterol. Thus, the term "hydrogenation product of plant sterols" as applied to plant stanols, and as used herein, includes not only the synthetic plant stanols but also those obtained from natural sources. Some plant stanols in this regard include sitostanol, campestanol, stigmastanol, spinostanol, taraxastanol, brassicastanol, desmostanol, chalinostanol, poriferastanol, clionastanol, and ergostanol. The skilled artisan can also select any plant stanol from those that are available. The invention also employs mixtures of plant stanols, such as two component, three component, and four component mixtures, as well as mixtures of plant sterols and plant stanols such as two component, three component, and four component mixtures.

Both the plant sterols and plant stanols include the various position isomers and stereo isomeric forms used in the art, such as the a and β isomers as well as plant sterols and plant stanols that contain small (from one to about four carbon atom) side chains. β-sitosterol and β-sitostanol respectively comprise one of the most effective plant sterols and one of the most effective plant stanols for lowering serum cholesterol in mammals.

In one embodiment of the invention, the mixing of the at least one hydrophobic plant sterol with the aqueous beverage concentrate to form a first dispersion of particles may be conducted at temperatures from about - 10°C to about 100°C (about 14 °F to about 212°F), or from about 0°C to about 82°C (about 32°F to about 180°F), or about 18°C to about 64°C (about 64°F to about 148°F), or about 24°C to about 57°C (about 75°F to about 135°F) for a period of time of from about 0.1 minutes to about 120 minutes, or from about 5 minutes to about 60 minutes, or from about 15 minutes to about 30 minutes, to form a first dispersion.

The apparatus employed for making the first dispersion of particles of the least one hydrophobic plant sterol and aqueous material, such as a beverage concentrate, comprises a high shear mixer (such as Arde-Barinco Model #CJ-4) or any large capacity (e.g., about 50 to about 300 gal.) high shear mixer. A commercial device for making the first dispersion comprises a "Liquiverter" (Trademark) manufactured under the trade name APV Liquiverter model 200 CLV, manufactured by APV, an Invensys Company.

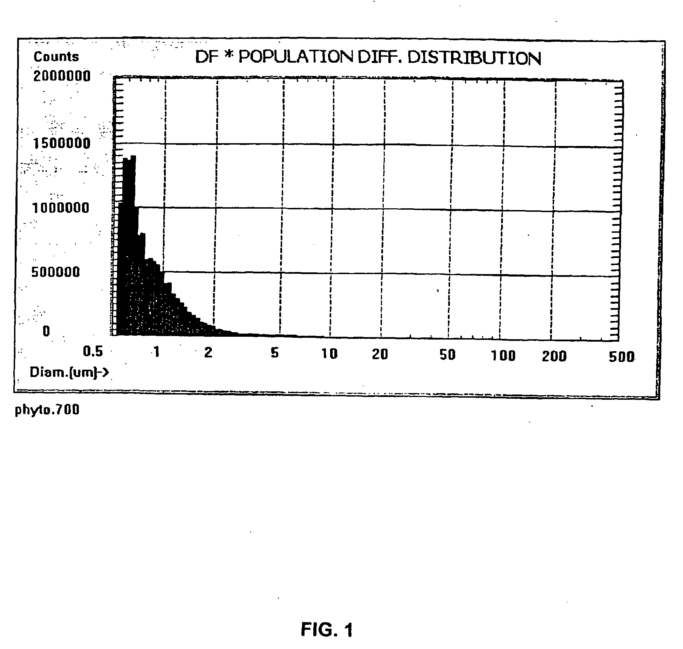

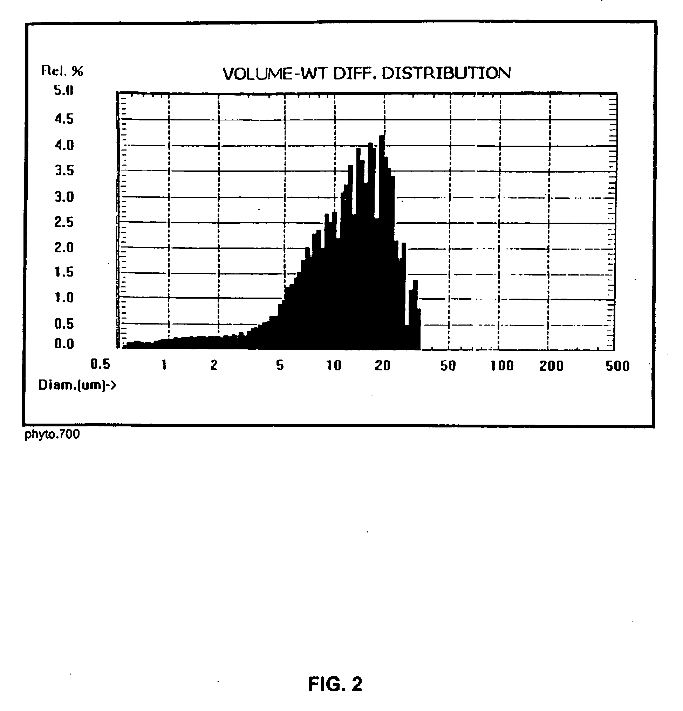

In another embodiment, the at least one plant sterol supplied may be micronized to a size of about 0.5 to about 10 microns. Fig. 1 shows an exemplary particle size distribution of the micronized plant sterol according to one embodiment of the present invention. In Fig. 1, "DF* Population Diff. Distribution" refers to the dilution factor multiplied by Population Differential Distribution, i.e., the number of counts in each channel had been multiplied by the factor that the sample was diluted, to yield the number of counts in the neat sample. Fig. 2 shows an exemplary volume distribution of the micronized plant sterol according to one embodiment of the present invention. In Fig. 2, "Volume-Wt-Diff. Distribution" refers to Volume Weighted Differential Distribution, i.e., taking all of the particles and looking at their volumes and making a determination of the contribution of each size channel to the overall volume of the sample. United States Patent No. 6,129,944 describes the method and apparatus used to manufacture the plant sterol composition of Figs. 1 and 2; however, the food industry also employs spray-drying techniques to form these compositions.

In Fig. 1, the numerical values for each of the reported "counts" (the values in the ordinate) and the numerical values for each of the reported particle diameters in microns (the values in the abscissa) can vary anywhere from plus or minus about 30% or plus or minus about 20% or plus or minus about 10% whereas in Fig. 2, the numerical values for each of the reported relative percentages (the values in the ordinate) and the numerical values for each of the reported particle diameters in microns (the values in the abscissa) can vary anywhere from plus or minus about 30% or plus or minus about 20% or plus or minus about 10%. Although the data of FIG. 1 and FIG. 2 refer to a specific plant sterol product, these data may also define the particle size and particle size distribution of any of the plant sterols employed according to the invention, such as those described in this specification, and those that are used in the art.

It is believed that in forming the first dispersion of the at least one hydrophobic plant sterol and aqueous material, the shear stress and/or shear rate applied to the hydrophobic plant sterol with the aqueous medium is sufficient to form a somewhat stable dispersion of particles of the at least one hydrophobic plant sterol and the aqueous material; however, the first dispersion does not have sufficient long term stability that would allow its use in consumer products such as juices, beverages, juice drinks, and the like.

The particle size of the at least one hydrophobic plant sterol of both the first dispersion and the second dispersion may substantially follow a bell curve particle size distribution well known to a person with ordinary skill in the art.

The aqueous material comprises water with additional compounds and compositions dissolved or dispersed in it, either as a dispersion of solids in water or an emulsion of a liquid in water or water in a liquid. This defines the aqueous material of the invention, prior to mixing it with the at least one hydrophobic plant sterol. When employing the aqueous material with a dissolved or dispersed compound or composition, the solids content of the aqueous material, such as an aqueous beverage concentrate is from about 200 grams per liter of the aqueous material to about 1000 grams per liter of the aqueous material, or about 400 grams per liter to about 900 grams per liter, or about 600 grams per liter to about 800 grams per liter. "Solids content," as that term applies to the "aqueous material" of the present invention, also includes any liquid added to the water used in forming an emulsion type of "aqueous material" as defined herein.

The at least one hydrophobic plant sterol may be present in the first dispersion and/or the second dispersion in an amount from about 1 gram to about 100 grams per liter or from about 10 grams to about 60 grams per liter, or about 20 grams to about 30 grams per liter of the aqueous material, concentrate, or beverage product. In one embodiment, the at least one hydrophobic plant sterol is present in the first dispersion and/or the second dispersion in an amount from about 15 grams to about 30 grams per liter of the aqueous material, concentrate, or beverage product.

In another embodiment, the process may also optionally comprise heating said second dispersion to form a heated second dispersion.

In another aspect, the first dispersion is cooled to a temperature from about 0°C to about 100°C (about 32°F to about 212°F), or about 13°C to about 87°C (about 55°F to about 189°F), or about 26°C to about 75°C ( about 78°F to about 167°F) for a period of time from about 1 second to about 30 seconds, or from about 2 seconds to about 10 seconds, or from about 5 seconds to about 7 seconds prior to homogenizing to form the second dispersion of particles of the at least one hydrophobic plant sterol and the aqueous material.

In a further embodiment, the first dispersion is cooled to a temperature of from about 22°C (about 72°F) to about 71°C (about 160°F) for a period of time of from about 1 second to about 12 seconds prior to the homogenizing.

In another embodiment of the present invention, the second dispersion is optionally heated to a temperature of from about 0°C (about 32°F) to about 100°C (about 212°F) for a period of time of from about 1 second to about 20 seconds to form a heated second dispersion.

In yet another embodiment, the second dispersion is optionally heated to a temperature from about 49°C (about 120°F) to about 88°C (about 190°F) for a period of time of from about 1 second to about 20 seconds to form a heated second dispersion.

In a further embodiment, the optionally heated second dispersion is cooled to a temperature from about -8°C to about 32°C (about 17°F to about 90°F), or about 2°C to about 4°C (about 35°F to about 40°F), for a period of time from about 1 second to about 12 seconds, or from about 3 seconds to about 7 seconds.

The homogenizing of the first dispersion to obtain a second dispersion of particles of the at least one hydrophobic plant sterol and the aqueous beverage concentrate is conducted in a homogenizer (such as APV model #APV 1000), which may function by forcing the dispersion through a small orifice at high pressures. The homogenizing may be carried out at a pressure from about 100 psi to about 14,500 psi, or 500 psi to about 10,000 psi, or 1,000 psi to about 5,000 psi (690 kPa to 100 MPa, or 3.5 MPa to 69 MPa, or 6.9 MPa to 35 MPa). In one embodiment, the homogenizing is carried out at a pressure of about 2,000 psi to about 5,000 psi (13.8 MPa to 35 MPa).

The invention also relates to conducting homogenizing at different pressures in single or multiple stages such as one stage, two stages, three stages, four stages or more.

Homogenization at high pressures and low pressures may also proceed, for example, according to any of the following parameters and combinations thereof:

| **High Pressure** | | **Low Pressure** | |
|---|---|---|---|
| about 2000 psi | (13.8 MPa) | about 300 psi | (2.1 MPa) |
| about 3000 psi | (20.6 MPa) | about 400 psi | (2.7 MPa) |
| about 3000 psi | (20.6 MPa) | about 500 psi | (3.5 MPa) |
| about 5000 psi | (35 MPa) | about 1000 psi | (6.9 MPa) |
| about 3400 psi | (23.4 MPa) | about 600 psi | (4.1 MPa) |

The sequence generally is to conduct the homogenization first at a high pressure than a low pressure, but the method of the invention also includes conducting the homogenization with different sequences of pressures, and in one embodiment, with more than one homogenizer.

Various beverage concentrates can be employed as the aqueous material according to the method of the invention, however, in one embodiment, the process involves producing a substantially stable dispersion comprising at least one hydrophobic plant sterol and an aqueous citrus juice concentrate such as an orange juice concentrate.

In its broader aspect, the aqueous material of the invention comprises water in combination with nutrients, flavorants, sweeteners, carbon dioxide and other gases, and combinations thereof. In another aspect the aqueous material is a concentrate of a fruit juice, or fruit flavor, such as citrus juices including orange, lemon, lime, tangerine, mandarin and, grapefruit juice, and other juice and fruit flavor concentrates such as acerola, grape, pear, passion fruit, pineapple, banana, apple, cranberry, cherry, raspberry, peach, plum, grape, currant, cranberry, blackberry, blueberry, strawberry, mirabelle, watermelon, honeydew, cantaloupe, mango, papaya, botanical flavors such as flavors derived from cola, tea, coffee, chocolate, vanilla, almond, vegetable juices and flavors such as tomato, cabbage, celery, cucumber, spinach, carrot, lettuce, watercress, dandelion, rhubarb, beet, cocona, guava, han guo, and mixtures thereof, such as two component, three component and four component mixtures.

The aqueous material of the invention may also comprise concentrates of typical sport beverages, and beverages used to treat loss of fluids due to illness, and which contain sucrose syrup, glucose-fructose syrup, citric acid, sodium citrate, monopotassium phosphate and potassium salts, and other materials for replenishing lost electrolytes, whether as a product requiring the addition of water or in admixture with water.

The concentrates of the present invention can be diluted with water to form juices or drinks. For example, where the concentrate includes a sugar or mixture of sugars, it can be diluted with water to about 2° Brix to about 20° Brix, or about 6° Brix to about 16° Brix, or about 11 ° Brix to about 13° Brix. The sugars employed according to the present invention may generally comprise carbohydrate materials such as fructose, sucrose, glucose and the like as well as the other sugars used in the art as described by McMurry, Organic Chemistry, Third Edition, pp. 916-950, Hawley's Condensed Chemical Dictionary, Twelfth Edition, p. 1100, and Hackh's Chemical Dictionary, Third Edition, pp. 815-817. Mixtures of sugars can also be used, such as two component, three component, or four component mixtures.

The process for producing a substantially stable dispersion comprising the at least one hydrophobic plant sterol and an aqueous material may also comprise adding at least one water soluble vitamin, such as vitamin C, vitamin B6 and/or vitamin B12, folic acid, and/or at least one oil soluble vitamins such as vitamin A, beta carotene, vitamin B, e.g., the D vitamins, vitamin E, and vitamin K, to the substantially stable dispersion, and any mixtures thereof, such as two component, three component and four component mixtures, either before, during or after the production of the substantially stable dispersion of the invention, e.g., by adding the vitamin or vitamins to the step for manufacturing the first dispersion or the step for manufacturing the second dispersion, or both such steps. The addition of a vitamin, such as vitamins B and E varies to obtain an RDA from about 1% to about 100%, or about 5 to about 30%, or about 15 to about 20% of the RDA for each vitamin per unit serving.

The following examples illustrate the invention.

### Example 1:

Combining the following components provided a base mixture of hydrophobic plant sterol with an aqueous material before subsequent processing to form a first dispersion.

The composition was formulated to obtain the following:

Base Ingredients

| | | |
|---|---|---|
| Desired Volume | 2.2 Gallons | (8.3 dm³) |
| Water | 2,079.4 grams | |
| Orange Concentrate | 7,837.0 grams | |
| Orange Flavor | 119:6 grams | |
| Orange Oil | 6.3 grams | |
| Plant.sterol | 198.1 grams | |
| Total | 10,240.8 grams | |

Finished Product Ingredients

| | | |
|---|---|---|
| Desired Volume | 4.4.gallons | (16.6 dm³) |
| Water | 13,262.9 grams | |
| Base | 4,178.3 grams | |

| **Base Specifications** | **OPT.** | **MIN.** | **MAX.** |
|---|---|---|---|
| Percent Soluble Solids | 49.7 | 49.4 | 50.5 |
| Refractometer °Brix | 49.8 | 49.5 | 50.6 |
| %Acid w/w as citric | 2.8 | 2.5 | 3.1 |
| °Brix/acid ratio | 17.5 | 15.7 | 19.8 |

The substantially stable dispersion of the oleophilic plant sterol and the orange juice concentrate as the aqueous material had a concentration of 48.9° Brix (refractometer Brix, corrected for acid).

The mixture was blended using an Arde-Barinco Model No. CJ-4 high shear mixer at 7000 rpm for about 15 minutes to produce a first dispersion having an average particle size of about 10 microns and a particle size distribution of about 0.5 microns to about 30 microns with the maximum particle size being about 30 microns.

Homogenizing the first dispersion in an APV homogenizer, Model No. APV 1000 from the APV Homogenizer Group (An Invensys Company) at 2,500 psi (17 MPa) and then at 500 psi (3.4 MPa) produced the second dispersion.

The second dispersion comprised a substantially stable dispersion consisting essentially of the hydrophobic plant sterol and the orange juice concentrate as the aqueous material. Adding water to the substantially stable dispersion produced an orange juice product of 12.0° Brix. The product was manufactured to the following specifications:

| **Product Specifications** | **OPT.** | **MIN.** | **MAX.** |
|---|---|---|---|
| Percent Soluble Solids | 12.0 | 11.9 | 12.2 |
| Refractometer °Brix | 11.9 | 11.8 | 12.1 |
| %Acid w/w as citric | 0.67 | 0.65 | 0.69 |
| ° Brix/acid ratio | 18.0 | 17.3 | 18.8 |

### Example 2:

Combining the following components provided a base mixture of hydrophobic plant sterol with an aqueous material before subsequent processing to form a first dispersion.

The composition was formulated to obtain the following:

Base Ingredients

| | | |
|---|---|---|
| Desired Volume | 0.8 Gallons | (3.0 dm³) |
| Water | 180.2 grams | |
| Orange Concentrate | 3,363.0 grams¹ | |
| Orange Flavor | 53.1 grams | |
| Orange Oil | 2.7 grams | |
| Plant sterol | 76.7 grams² | |
| Total | 3.675.5 grams | |

| | | |
|---|---|---|
| ¹ Refractometer °Brix, 65 (corrected for acid); acid, 3.71 % (wt./wt.). ² ADM 09/2001 consisting essentially of betasitosterol, betasitostanol, campesterol, campestanol, stigmasterol, spinosterol, avenasterol, or brassicasterol having a particle size of from about 0.5 microns to about 30 microns. | | |

Finished Product Ingredients

| | | |
|---|---|---|
| Desired Volume | 4.8 gallons | (18.2 dm³) |
| Water | 4.1 gallons | (15.5 dm³) |
| Base | 0.8 gallons | (3.0 dm³) |

| **Base Specifications** | **OPT.** | **MIN.** | **MAX.** |
|---|---|---|---|
| Percent Soluble Solids | 61.6 | 61.1 | 62.4 |
| Refractometer °Brix | 61.6 | 60.7 | 62.0 |
| %Acid w/w as citric | 3.4 | 3.1 | 3.7 |
| °Brix/acid ratio | 18.0 | 16.4 | 20.0 |

The substantially stable dispersion of the oleophilic plant sterol and the orange juice concentrate as the aqueous material had a concentration of 61.2° Brix (refractometer Brix, corrected for acid).

The mixture was stirred using an Arde-Barinco Model No. CJ-4 high shear mixer at 7000 rpm for about 15 minutes and heated to 82.2°C (180°F) in 8 seconds and chilled to about 43.3°C to about 60°C (about 110°F to about 140°F) in about 5 seconds to produce a first dispersion having an average particle size of about 10 microns and a particle size distribution of about 0.5 microns to about 30 microns with the maximum particle size being about 30 microns.

Homogenizing the first dispersion in an APV homogenizer, Model No. APV 1000 from the APV Homogeniser Group (An Invensys Company) at 60°C (140°F) at 3,400 psi (23.4 MPa) and then at 600 psi (4.1 MPa) produced the second dispersion.

The second dispersion comprised a substantially stable dispersion consisting essentially of the hydrophobic plant sterol and the orange juice concentrate as the aqueous material. Adding water to the substantially stable dispersion produced an orange juice product of 12.0° Brix. The product was manufactured to the following specifications:

| **Product Specifications** | **OPT.** | **MIN.** | **MAX.** |
|---|---|---|---|
| Percent Soluble Solids | 12.0 | 11.9 | 12.2 |
| Refractometer °Brix | 11.9 | 11.8 | 12.1 |
| %Acid w/w as citric | 0.67 | 0.65 | 0.69 |
| °Brix/acid ratio | 18.0 | 17.3 | 18.8 |

### Example 3:

Combining the following components provided a base mixture of hydrophobic plant sterol with an aqueous material before subsequent processing to form a first dispersion.

The composition was formulated to obtain the following:

Base Ingredients

| | | |
|---|---|---|
| Desired Volume | 2,000.0 Gallons | (7.6 m³) |
| Water | 4,158.8 pounds | (1886 kg) |
| Orange Concentrate | 15,674.0 pounds | (7110 kg) |
| Orange Flavor | 239.2 pounds | (108.5 kg) |
| Orange Oil | 12.7 pounds | (5.8 kg) |
| Plant sterol | 396.2 pounds | (179.7 kg) |
| Total | 20,480.9 pounds | (9290 kg) |

Finished Product Ingredients

| | | |
|---|---|---|
| Desired Volume | 1,000 gallons | (3.8 m³) |
| Water | 6,631.5 pounds | (2885 kg) |
| Base | 2,089.1 pounds | (947.6 kg) |

| **Base Specifications** | **OPT.** | **MIN.** | **MAX.** |
|---|---|---|---|
| Percent Soluble Solids | 49.7 | 49.4 | 50.5 |
| Refractometer °Brix | 49.8 | 49.5 | 50.6 |
| %Acid w/w as citric | 2.8 | 2.5 | 3.1 |
| °Brix/acid ratio | 17.5 | 15.7 | 19.8 |

The substantially stable dispersion of the oleophilic plant sterol and the orange juice concentrate as the aqueous material had a concentration of 50.1° Brix (refractometer Brix, corrected for acid).

The mixture was blended in a 2200 gallon (8.3 m³) batch tank with constant agitation prior to adding the plant sterols or stanols. The dispersion was then pumped through a 200 gallon (0.75 m³) Norman Machinery Co. Model DS 200 high shear mixer and the plant sterols were added gradually to the high shear mixer through a ½ inch (1.3 cm) mesh screen to produce a first dispersion having an average particle size of about 10 microns and a particle size distribution of about 0.5 microns to about 30 microns with the maximum particle size being about 30 microns.

Homogenizing the first dispersion through a 30 gallon (113 dm³) per minute APV homogenizer at 2500 psi (17 MPa) and then at 500 psi (3.4 MPa) produced the second dispersion. This second dispersion was then heated to 72.7°C (163°F) in 8 seconds and chilled to about 2°C to about 4°C (about 35°F to about 40°F) in about 5 seconds.

The second dispersion comprised a substantially stable dispersion consisting essentially of the hydrophobic plant sterol and the orange juice concentrate as the aqueous material. Adding water to the substantially stable dispersion produced an orange juice product of 12.0° Brix. The product was manufactured to the following specifications:

| **Product Specifications** | **OPT.** | **MIN.** | **MAX.** |
|---|---|---|---|
| Percent Soluble Solids | 12.0 | 11.9 | 12.2 |
| Refractometer °Brix | 11.9 | 11.8 | 12.1 |
| %Acid w/w as citric | 0.67 | 0.05 | 0.69 |
| ° Brix/acid ratio | 18.0 | 17.3 | 18.8 |

The principles, various embodiments, and modes of operation of the present invention have been described in the foregoing written description.
All quantities expressed in percentages are percentages by weight, unless otherwise indicated.

## Claims

1. A process for producing a substantially stable dispersion comprising from 1 to 100 grams per liter of at least one hydrophobic plant sterol and an aqueous material comprising at least one fruit juice or fruit juice concentrate, wherein the solids content of said aqueous material is from 200 to 1000 grams per liter and wherein said plant sterol does not separate from said dispersion over a period of time from formation of said dispersion to 12 months after formation, after subjecting the dispersion to heating and cooling cycles and/or shelf storage during this time, comprising:
mixing the at least one hydrophobic plant sterol with said aqueous material to form a first dispersion of particles of the at least one hydrophobic plant sterol and said aqueous material; and
homogenizing the first dispersion to obtain a second dispersion of particles of the at least one hydrophobic plant sterol and said aqueous material, wherein the particle size of said hydrophobic plant sterol particles in said first dispersion is from 0.1 microns to 100 microns, or the particle size of said hydrophobic plant sterol particles in said second dispersion is from 0.1 microns to 100 microns, or wherein the particle size of said hydrophobic plant sterol particles in both said first dispersion and said second dispersion is from 0.1 microns to 100 microns,
wherein said first dispersion is not heated prior to homogenizing, and
with the proviso that said substantially stable dispersion does not contain any added emulsifiers or thickening agents.

2. The process of Claim 1 wherein said particle size of the hydrophobic plant sterol in said first dispersion is from 0.1 micron to 50 microns, preferably from 0.1 micron to 30 microns, more preferably from 0.1 micron to 10 microns, or
the particle size of the hydrophobic plant sterol in said second dispersion is from 0.1 micron to 50 microns, preferably from 0.1 micron to 30 microns, more preferably from 0.1 micron to 10 microns, or
the particle size of the hydrophobic plant sterol in at least one of the first and second dispersions is from 0.1 micron to 50 microns, preferably from 0.1 micron to 30 microns, more preferably from 0.1 micron to 10 microns.

3. The process of Claim 1 wherein greater than 50% of the hydrophobic plant sterol ranges in particle size from 0.2 microns to 10 microns, preferably from 0.2 microns to 2.5 microns, more preferably from 0.4 microns to 1.5 microns, even more preferably from 0.3 microns to 0.4 microns.

4. The process of Claim 1 wherein the at least one hydrophobic plant sterol is selected from:
sitosterol, campesterol, stigmasterol, spinosterol, taraxasterol, brassicasterol, desmosterol, chalinosterol, poriferasterol, clionasterol, and ergosterol.

5. The process of Claim 1 wherein the at least one hydrophobic plant sterol is selected from hydrogenation products of plant sterols.

6. The process of Claim 1 wherein the at least one hydrophobic plant sterol is selected from:
sitostanol, campestanol, stigmastanol, spinostanol, taraxastanol, brassicastanol, desmostanol, chalinostanol, poriferastanol, clionastanol, and ergostanol.

7. The process of Claim 1 wherein the solids content of said aqueous material is from 400 to 900 grams per liter, more preferably from 600 to 800 grams per liter of said aqueous material.

8. The process of Claim 1 wherein, in said first dispersion, the at least one hydrophobic plant sterol is present in an amount from 10 to 60 grams per liter, preferably from 15 to 30 grams per liter of the aqueous material.

9. The process of Claim 1 wherein said homogenizing is carried out at a pressure of from 0.7 MPa (100 psi) to 100 MPa (14,500 psi), preferably from 3.4 MPa (500 psi) to 69 MPa (10,000 psi), more preferably from 7 MPa (1,000 psi) to 34.4 MPa (5,000 psi), or even more preferably from 13.8 MPa (2,000 psi) to 34.4 MPa (5,000 psi).

10. The process of Claim 1 wherein said homogenizing is carried out in multiple stages, at different pressures, such as a first homogenizing at from 13.8 MPa (2000 psi) to 34,4 MPa (5000 psi) followed by a second homogenizing at a pressure of from 2 MPa (300 psi) to 7 MPa (1000 psi).

11. The process of Claim 1 wherein said aqueous material comprises at least one citrus juice concentrate.

12. The process of Claims 1 or 11 wherein water is added to said second dispersion of particles of the at least one hydrophobic plant sterol and said fruit juice or said citrus juice concentrate to obtain an aqueous beverage mixture.

13. The process of Claim 12 wherein the aqueous beverage mixture has a concentration of from 11° Brix to 13° Brix.

14. The process of Claim 11 wherein the at least one citrus juice concentrate is orange juice concentrate.

15. The process of Claim 1 further comprising adding at least one vitamin either before, during, or after the production of the substantially stable dispersion.

16. The process of Claim 15 wherein the at least one vitamin is chosen from water soluble vitamins and oil soluble vitamins.

17. The process of Claim 1 further comprising:
heating the second dispersion of particles of the at least one hydrophobic plant sterol and said aqueous material to produce a heated second dispersion.

18. The process of Claim 17 wherein said second dispersion of particles of the at least one hydrophobic plant sterol is heated to a temperature of from 0°C (32°F) to 100°C (212°F) for a period of time of from 1 second to 20 seconds, preferably from 49°C (120°F) to 88°C (190°F) for a period of time of from 1 second to 20 seconds.

19. The process of Claim 17 wherein said heated second dispersion is cooled to a temperature ranging from -8°C (17°F) to 32°C (90°F) for a period of time of from 1 second to 12 seconds, preferably from 2°C (35°F) to 4°C (40°F) for a period of time of 3 seconds to 7 seconds.

## Patentansprüche

1. Verfahren zum Erzeugen einer im Wesentlichen stabilen Dispersion, die 1 bis 100 Gramm pro Liter wenigstens eines hydrophoben Pflanzenstyrols und ein wässriges Material mit wenigstens einem Fruchtsaft oder Fruchtsaftkonzentrat enthält, wobei der Feststoffanteil des wässrigen Materials zwischen 200 und 1.000 Gramm pro Liter beträgt, wobei sich das Pflanzenstyrol nach der Anwendung von Erhitzungs- und Abkühlungszyklen auf die Dispersion und / oder Regallagerung während dieser Zeit von der Dispersion für einen Zeitraum ab der Bildung der Dispersion bis zu 12 Monaten nach der Bildung nicht trennt und wobei das Verfahren die folgenden Schritte umfasst:
- Mischen des wenigstens einen hydrophoben Pflanzenstyrols mit dem wässrigen Material, um eine ersten Dispersion von Partikeln des wenigstens einen hydrophoben Pflanzenstyrols und des wässrigen Materials zu bilden, und
- Homogenisieren der ersten Dispersion zum Bilden einer zweite Dispersion von Partikeln des wenigstens einen hydrophoben Pflanzenstyrols und eines wässrigen Materials, wobei die Partikel der hydrophoben Pflanzenstyrolpartikel in der ersten Dispersion eine Partikelgröße zwischen 0,1 µm und 100µm aufweisen oder die hydrophoben Pflanzenstyrolpartikel der zweiten Dispersion eine Partikelgröße zwischen 0,1µm und 100µm aufweisen oder wobei die hydrophoben Pflanzenstyrolpartikel in der ersten und der zweiten Dispersion eine Partikelgröße zwischen 0,1 µm und 100µm aufweisen,
wobei die erste Dispersion vor dem Homogenisieren nicht erhitzt wird und wobei die im Wesentlichen stabile Dispersion keinerlei Emulgatoren oder Verdickungsmittel enthält.

2. Verfahren nach Anspruch 1, wobei die Partikel des hydrophoben Pflanzenstyrols der ersten Dispersion eine Größe zwischen 0,1µm und 50µm, vorzugsweise zwischen 0,1µm und 30µm, weiter bevorzugt zwischen 0,1µm und 10µm, oder
die Partikel des hydrophoben Pflanzenstyrols der zweiten Dispersion eine Größe zwischen 0,1µm und 50µm, vorzugweise zwischen 0,1µm und 30µm, weiter bevorzugt zwischen 0,1µm und 10µm, oder
die Partikel des hydrophoben Pflanzenstyrols in wenigsten der ersten oder wenigstens der zweiten Dispersion, eine Größe zwischen 0,1µm und 50µm, vorzugsweise zwischen 0,1µm und 30µm, weiter bevorzugt zwischen 0,1µm und 10µm aufweisen.

3. Verfahren nach Anspruch 1, wobei mehr als 50% der Partikel des hydrophoben Pflanzenstyrols eine Größe im Bereich zwischen 0,2µm und 10µm, vorzugsweise zwischen 0,2µm und 2,5µm, weiter bevorzugt zwischen 0,4µm und 1,5µm, noch weiter bevorzugt zwischen 0,3µm und 0,4µm aufweisen.

4. Verfahren nach Anspruch 1, wobei das wenigstens eine hydrophobe Pflanzenstyrol ausgewählt ist aus: Sitosterol, Campesterol, Stigmasterol, Spinosterol, Taraxasterol, Brassicasterol, Desmosterol, Chalinosterol, Poriferasterol, Clionasterol und Ergosterol.

5. Verfahren nach Anspruch 1, wobei das wenigstens eine hydrophobe Pflanzenstyrol aus Hydrierungsprodukten von Pflanzenstyrolen ausgewählt ist.

6. Verfahren nach Anspruch 1, wobei das wenigstens eine hydrophobe Pflanzenstyrol ausgewählt ist aus: Sitosterol, Campesterol, Stigmasterol, Spinosterol, Taraxasterol, Brassicasterol, Desmosterol, Chalinosterol, Poriferasterol, Clionasterol und Ergosterol.

7. Verfahren nach Anspruch 1, wobei der Feststoffanteil des wässrigen Materials zwischen 400 und 900 Gramm pro Liter, vorzugweise zwischen 600 und 800 Gramm pro Liter des wässrigen Materials beträgt.

8. Verfahren nach Anspruch 1, wobei in der ersten Dispersion der Anteil des wenigstens einen hydrophoben Pfalnzenstyrols zwischen 10 und 60 Gramm pro Liter, vorzugsweise zwischen 15 und 30 Gramm pro Liter des wässrigen Materials beträgt.

9. Verfahren nach Anspruch 1, wobei das Homogenisieren bei einem Druck zwischen 0,7 MPa (100 psi) und 100 MPa (14.500 psi), vorzugweise zwischen 3,4 MPa (500 psi) und 69 MPa (10.000 psi), weiter bevorzugt zwischen 7 MPa (1.000 psi) und 34,4 MPa (5.000 psi), noch weiter bevorzugt zwischen 13,8 MPa (2.000 psi) und 34,4 MPa (5.000 psi) durchgeführt wird.

10. Verfahren nach Anspruch 1, wobei das Homogenisieren in mehreren Stufen bei unterschiedlichen Drücken durchgeführt wird, beispielsweise einem ersten Homogenisieren zwischen 13,8 MPa (2.000 psi) und 34,4 MPa (5.000 psi) gefolgt von einem zweiten Homogenisieren bei einem Druck zwischen 2 MPa (300 psi) und 7 MPa (1.000 psi).

11. Verfahren nach Anspruch 1, wobei das wässrige Material mindestens ein Zitrussaftkonzentrat enthält.

12. Verfahren nach Anspruch 1 bis 11, wobei zur zweiten Dispersion von Partikeln des wenigstens einen hydrophoben Pflanzenstyrols Wasser zugegeben wird und der Fruchtsaft oder das Zitrussaftkonzentrat aufkonzentriert wird, um eine wässrige Getränkemischung zu bilden.

13. Verfahren nach Anspruch 12, wobei die wässrige Getränkemischung eine Konzentration zwischen 11° Brix und 13° Brix aufweist.

14. Verfahren nach Anspruch 11, wobei wenigstens eines der Zitrussaftkonzentrate Orangensaftkonzentrat ist.

15. Verfahren nach Anspruch 1, wobei wenigstens ein Vitamin vor, während oder nach dem Erzeugen der im Wesentlichen stabilen Dispersion zugegeben wird.

16. Verfahren nach Anspruch 15, wobei wenigstens eines der Vitamine aus wasserlöslichen Vitaminen oder öllöslichen Vitaminen ausgewählt ist.

17. Verfahren nach Anspruch 1, wobei das Verfahren den folgenden Schritt aufweist:
Aufheizen der zweiten Dispersion von Partikeln des wenigstens einen hydrophoben Pflanzenstyrols und des wässrigen Materials, um eine erhitzte zweite Dispersion zu erzeugen.

18. Verfahren nach Anspruch 17, wobei die zweite Dispersion von Partikeln des wenigstens einen hydrophoben Pflanzenstyrols auf eine Temperatur zwischen 0°C (32°F) und 100°C (212°F) für einen Zeitraum zwischen 1 Sekunde und 20 Sekunden, vorzugsweise eine Temperatur zwischen 49°C (120°F) und 88°C (190°F) für einen Zeitraum zwischen 1 Sekunde und 20 Sekunden erhitzt wird.

19. Verfahren nach Anspruch 17, wobei die erhitzte zweite Dispersion auf eine Temperatur zwischen -8°C (17°F) und 32°C (90°F) für einen Zeitraum zwischen 1 Sekunde und 12 Sekunden, vorzugsweise eine Temperatur zwischen 2°C (35°F) und 4°C (40°F) für einen Zeitraum zwischen 3 Sekunden und 7 Sekunden gekühlt wird.

## Revendications

1. Procédé pour produire une dispersion sensiblement stable comprenant de 1 à 100 g/l d'au moins un stérol végétal hydrophobe et un produit aqueux comprenant au moins un jus de fruits ou un concentré de jus de fruits, où la teneur en solides dudit produit aqueux est de 200 à 1 000 g/l et où ledit stérol végétal ne se sépare pas de ladite dispersion pendant une durée allant de la formation de ladite dispersion à 12 mois après la formation, après exposition de la dispersion à des cycles de chauffage et de refroidissement et/ou un stockage pendant cette durée, comprenant :
le mélange du au moins un stérol végétal hydrophobe avec ledit produit aqueux pour former une première dispersion de particules du au moins un stérol végétal hydrophobe et dudit produit aqueux ; et
l'homogénéisation de la première dispersion pour obtenir une seconde dispersion de particules du au moins un stérol végétal hydrophobe et dudit produit aqueux, où la taille de particules desdites particules de stérol végétal hydrophobe dans ladite première dispersion est de 0,1 µm à 100 µm, ou bien la taille de particules desdites particules de stérol végétal hydrophobe dans ladite seconde dispersion est de 0,1 µm à 100 µm, ou bien où la taille de particules desdites particules de stérol végétal hydrophobe dans ladite première dispersion et ladite seconde dispersion est de 0,1 µm à 100 µm,
où ladite première dispersion n'est pas chauffée avant l'homogénéisation, et
avec la condition que ladite dispersion sensiblement stable ne contienne aucun émulsifiant ou agent épaississant ajouté.

2. Procédé selon la revendication 1 où ladite taille de particules du stérol végétal hydrophobe dans ladite première dispersion est de 0,1 µm à 50 µm, de préférence de 0,1 µm à 30 µm, de préférence encore de 0,1 µm à 10 µm, ou bien
la taille de particules du stérol végétal hydrophobe dans ladite seconde dispersion est de 0,1 µm à 50 µm, de préférence de 0,1 µm à 30 µm, de préférence encore de 0,1 µm à 10 µm, ou bien
la taille de particules du stérol végétal hydrophobe dans au moins l'une des première et seconde dispersions est de 0,1 µm à 50 µm, de préférence de 0,1 µm à 30 µm, de préférence encore de 0,1 µm à 10 µm.

3. Procédé selon la revendication 1 où plus de 50 % du stérol végétal hydrophobe ont une taille de particules allant de 0,2 µm à 10 µm, de préférence de 0,2 µm à 2,5 µm, de préférence encore de 0,4 µm à 1,5 µm, de préférence encore de 0,3 µm à 0,4 µm.

4. Procédé selon la revendication 1 où le au moins un stérol végétal hydrophobe est choisi parmi :
le sitostérol, le campestérol, le stigmastérol, le spinostérol, le taraxastérol, le brassicastérol, le desmostérol, le chalinostérol, le poriférastérol, le clionastérol et l'ergostérol.

5. Procédé selon la revendication 1 où le au moins un stérol végétal hydrophobe est choisi parmi les produits d'hydrogénation de stérols végétaux.

6. Procédé selon la revendication 1 où le au moins un stérol végétal hydrophobe est choisi parmi :
le sitostanol, le campestanol, le stigmastanol, le spinostanol, le taraxastanol, le brassicastanol, le desmostanol, le chalinostanol, le poriférastanol, le clionastanol et l'ergostanol.

7. Procédé selon la revendication 1 où la teneur en solides dudit produit aqueux est de 400 à 900 g/l, de préférence encore de 600 à 800 g/l dudit produit aqueux.

8. Procédé selon la revendication 1 où, dans ladite première dispersion, le au moins un stérol végétal hydrophobe est présent en une quantité de 10 à 60 g/l, de préférence de 15 à 30 g/l du produit aqueux.

9. Procédé selon la revendication 1 où ladite homogénéisation est conduite à une pression de 0,7 MPa (100 psi) à 100 MPa (14 500 psi), de préférence de 3,4 MPa (500 psi) à 69 MPa (10 000 psi), de préférence encore de 7 MPa (1 000 psi) à 34,4 MPa (5 000 psi), ou de préférence encore de 13,8 MPa (2 000 psi) à 34,4 MPa (5 000 psi).

10. Procédé selon la revendication 1 où ladite homogénéisation est conduite dans des stades multiples, à des pressions différentes, comme une première homogénéisation à 13,8 MPa (2 000 psi) à 34,4 MPa (5 000 psi) suivie par une seconde homogénéisation à une pression de 2 MPa (300 psi) à 7 MPa (1 000 psi).

11. Procédé selon la revendication 1 où ledit produit aqueux comprend au moins un concentré de jus d'agrumes.

12. Procédé selon la revendication 1 ou 11 où de l'eau est ajoutée à ladite seconde dispersion de particules du au moins un stérol végétal hydrophobe et dudit jus de fruits ou dudit concentré de jus d'agrumes pour obtenir un mélange de type boisson aqueuse.

13. Procédé selon la revendication 12 où le mélange de type boisson aqueuse a une concentration de 11° Brix à 13° Brix.

14. Procédé selon la revendication 11 où le au moins un concentré de jus d'agrumes est un concentré de jus d'orange.

15. Procédé selon la revendication 1 comprenant en outre l'addition d'au moins une vitamine avant, pendant ou après la production de la dispersion sensiblement stable.

16. Procédé selon la revendication 15 où la au moins une vitamine est choisie parmi les vitamines solubles dans l'eau et les vitamines solubles dans l'huile.

17. Procédé selon la revendication 1 comprenant en outre :
le chauffage de la seconde dispersion de particules du au moins un stérol végétal hydrophobe et dudit produit aqueux pour produire une seconde dispersion chauffée.

18. Procédé selon la revendication 17 où ladite seconde dispersion de particules du au moins un stérol végétal hydrophobe est chauffée à une température de 0°C (32°F) à 100°C (212°F) pendant une durée de 1 s à 20 s, de préférence de 49°C (120°F) à 88°C (190°F) pendant une durée de 1 s à 20 s.

19. Procédé selon la revendication 17, où ladite seconde dispersion chauffée est refroidie à une température allant de -8°C (17°F) à 32°C (90°F) pendant une durée de 1 s à 12 s, de préférence de 2°C (35°F) à 4°C (40°F) pendant une durée de 3 s à 7 s.
